(19) Europäisches Patentamt
European Patent Office
Office européen des brevets

(11) Publication number: **0 214 254 B1**

(12) # EUROPEAN PATENT SPECIFICATION

(45) Date of publication of patent specification: **17.06.92**

(51) Int. Cl.5: **A61K 7/06**, A61K 7/48, A61K 31/405

(21) Application number: **86901842.4**

(22) Date of filing: **03.03.86**

(86) International application number: **PCT/EP86/00108**

(87) International publication number: **WO 86/05093 (12.09.86 86/20)**

The file contains technical information submitted after the application was filed and not included in this specification

(54) **MELATONIN COMPOSITIONS AND USES THEREOF.**

(30) Priority: **04.03.85 GB 8505537**
**27.08.85 US 770054**

(43) Date of publication of application: **18.03.87 Bulletin 87/12**

(45) Publication of the grant of the patent: **17.06.92 Bulletin 92/25**

(84) Designated Contracting States: **AT BE CH DE FR GB IT LI LU NL SE**

(56) References cited:
**EP-A- 126 630**

**Merck Index (1983), p. 5633, ri.col.**

**C.A.96 (1982), No. 156305w**

**See also references of WO8605093**

(73) Proprietor: **CELLENA (CELL ENGENEERING) A.G.**
**Lohwisstrasse, 12**
**CH-8123 Ebmatingen(CH)**

(72) Inventor: **PIERPAOLI, Walter**
**Lohwisstrasse, 12**
**CH-8123 Ebmatingen(CH)**
Inventor: **REGELSON, William**
**1402 Confederate Avenue**
**Richmond, VA 23227(US)**

(74) Representative: **Gutmann, Ernest et al**
**S.C. Ernest Gutmann - Yves Plasseraud 67, boulevard Haussmann**
**F-75008 Paris(FR)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 214 254 B1

## Description

The present invention primarily relates to a topical composition for treating the skin and/or scalp of a human host to improving its cosmetic and physical appearance as defined in the claims. More particularly, the present invention relates to the topical treatment of specific conditions of the skin and scalp of a human host so as to enhance the condition of the skin to assist in the rejuvenation of partially degenerated hair follicles by using melatonin compounds as defined below.

Even more particularly, the present invention relates to a composition for enhancing skin condition and tone, the prevention or amelioration of the conditions or symptoms of acne, seborrhea, and for the rejuvenation of partially degenerated hair follicles through the use of melatonin compounds, homologues or derivatives.

### Background of The Invention

Melatonin is a hormone secreted by the pineal gland that controls seasonal and circadian hormonal rhythms, and alters the metabolism of testosterone and enhances the availability of estrogen receptors in target tissues.

Houssay et al (1966) (1) have shown that the pineal gland and the parenteral administration of melatonin acts on the skin of mice to slow hair growth waves in mice.

Houssay et al (1966) (2) describe that the parenteral treatment of mice with melatonin effects the hair growth waves in mice.

Rose et al (1984) have shown that it is possible to induce the growth of winter pelage in mink by implanting melatonin.

Logan et al (1980) have found that melatonin can inhibit in vitro melanogenesis in hair follicles of the Siberian hamster.

Heath et al (1982) have shown that follicular development was blocked in mice injected with mela tonin.

Rats were used in the assay to measure effects on LH release and ovulation. Melatonin analogs were given p.o. and intravenously. Pronounced increase in activity and half-life was noted with halogenation on the 6 position.

Melatonin and related metabolites were found to be the principal excretory products of the pineal gland (epiphysis cereberi), an endocrine organ present intracranially in all vertebrates (Reiter, 1983).

Melatonin (N-acetyl-methoxytryptamine) is primarily derived from tryptophan and synthesized via the action of tryptophane hydroxylase (Lovenberg et al., 1967). The pineal gland is the primary place for a major portion of physiological indolamine metabolism including that of the neuroeffector serotonin which is a major source of melatonin synthesis.

Melatonin synthesis is governed by light exposure and in man and other mammals and primarily produced in conjunction with night or in darkness from its pineal endocrine source. In addition to the pineal body, both the retina, the harderian gland (in rodents) and gastrointestinal tract are producers of melatonin (Ralph, 1981; Reiter et al, 1983: Raikhlin et al., 1975).

An example of systemic melatonin effects on reproductive hormonal cycling are seen in the depression of testosterone production in mice given melatonin (Petterborg & Reiter, 1981). Alternatively, depending on species, testicular regression can be prevented and testosterone activity maintained (Turek, 1977; Stetson et al 1983) in hamsters. Melatonin, given by injection, can alter estrous cycling in female rats (Trentini, et al., 1980). Evidence supports the possibility that melatonin levels may be a factor in suppression of puberty in man (Tamarkin et al., 1985).

Melatonin has been given at dosages of 1mg/kg i.m. with advanced breast cancer for periods up to 2 months. These studies were conducted after trial at 5 and 20mg/kg i.m. daily for up to 10 days in monkeys with no report on clinical response other than a report of a decline in urinary estrogen (Burns, 1973). Blask (1984) has reviewed the role of melatonin in the clinical treatment of malignancy. He cites DiBella and Starr as achieving inhibitory clinical results in a variety of tumors.

In regard to local modulation and inhibition of steroid synthesis, melatonin on in vivo and in vitro treatment has shown in vivo and in vitro inhibition of testicular synthesis from cholesterol and pregnenolone precursors of testosterone and androstenedione synthesis in the rat testes (Peat & Kinson, 1971).

Orally administered, melatonin was found to lower ventral prostate and seminal vesicle weight and the 3/beta-hydroxysteroid oxidoreductase was increased but not the 5 alpha reductase in the ventral prostate and seminal vesicles of pinealectomized rats (Horst et al., 1982). The authors felt that this reflects on increased androgenic catabolism resulting in prostatic involution. The effects of melatonin on prostatic androgen receptors can depend on the age of the animal and light cycle exposure (Moeller et al., 1983).

Melatonin in vitro when combined with chorinonic gonadotrophin or ovine luteinizing hormone increased the secretion of estrogens and progesterone in isolated granulosa cells of the rat. Melatonin, in relation to ovarian function, showed a progonadal trophic effect (Fiske et al., 1984).

In regard to local stimulation of estrogen receptor availability by melatonin in cutaneous areas of androgenic and estrogenic hormone sensitivity. There is evidence that melatonin increases cytoplasmic estrogen receptor activity in hamster uteri and similar effects have been observed in estrogen receptor binding activity in human breast cancer cells (Danforth et al., 1983).

Other reviews of the physiological role of melatonin are found in:

- G.M. VAUGHN et al., titled "Evidence for a pineal-gonad relationship in the human", published in Prog. Reprod. Biol Vol. 4, p. 191-223, 1978.
- H.L. JUDD, titled "Biorhythms of gonadotrophins and testicular hormone secretion", published in Endocrine Rhythms, 1979.
- D.P. CARDINALI et al., titled "Melatonin action: sites and possible mechanisms in brain", published in "The pineal gland and its endocrine role", J. AXELROD, F. FRASCHINI and G.P. VELO, eds. Proc. Nato Adv. Study, Erice, Italy, p. 551-575, Plenum Press, New York, 1982.
- R.J. WURTMAN, et al., titled "The secretion and effects of melatonin in humans", published in "The pineal gland and its endocrine role", J. AXELROD, R. FRASCHINI and G.P. VELO, ed. Proc. nato Avd., Erice, Italy, p. 551-575, Plenum Press, New York, 1982.
- The Third Colloquium of the European Pineal Study Group, PECS 1984, published in EPSG Newsletter of August, 1984.
- R.J. Wurtman et al, entitled "Physiological Control of Melantonin Synthesis and Secretion: Mechanisms Generating Rhythms in Melatonin, Methoxytryptophol, and Arginine Vasotocin Levels and Effects on the Pineal of Endogenous Catecholamines, the Estrous Cycle, and Environmental Lighting", J. of Neural Transmission, Suppl. 13, 59-70 (1978).
- Ivor Smith, entitled "Indoles of Pineal Origin: Biochemical and Physiological Status", Psychoneuroendocrinology, Vol. 8, No. 1, pp 41-60 (1983).

Surprisingly, none of the prior art studies are concerned with the dermatological effects of the administration of melatonin to a human host. Moreover, there have been no previous studies regarding the topical application of melatonin, its homologues or derivatives for humans. The prior art studies do indicate that the compounds of the invention can be safely administered to humans in the treatment of various diseases.

## Summary Of The Invention

In accordance with the present invention, there is provided a composition for treating the skin and scalp of a mammal by the administration of a composition containing a melatonin compound as defined in the claims in order to improve the cosmetic, physical appearance or functional role.

More particularly, the present invention provides a topical composition for use in the treatment of the skin and/or scalp of a human host in order to enhance the skin's condition and tone, prevent or ameliorate the conditions or symptoms of acne, seborrhea, and for the rejuvention of live but degenerated hair follicles and provides the use of compounds as defined below for the manufacture of those compositions for the above treatments.

The preferred active principle of the topical compositions of the invention consists of melatonin itself. Among the chemical homologues useful for the production of compositions according to the invention, there can be mentioned the class of compounds selected from the class consisting of melatonin, 5-methoxytryptamine, 5-methoxytryptophan, 5-methoxytryptophol, 5-methoxyindole-3-acetic acid and 6-hydroxy-melatonin.

The most preferred active principle is melatonin itself, the formula of which is :

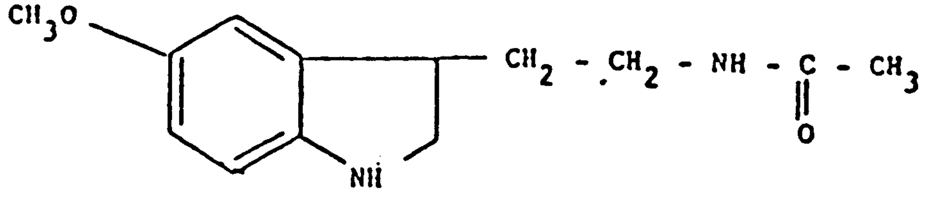

These compounds can be obtained by synthetic processes, general methods of manufacture which can

be derived from those published by J. SZMUSZKOVICZ "Synthesis of N-acetyl-5-methoxy-tryptamine", J. Org. Chem. 25, 857 (1960),J. SUPNIEWSKI et al., "Synthesis of melatonin (5-methoxy-N-acetyltryptamine", published in Bull. Acad. Polon. Sci. Ser. Biol., 8, p. 479-481, 1960; or MASHKOVSKY et al. in Farmakol. Toksikol., 26, n 1, 10, 1963, said methods being of course in each case adapted to the particular compound sought.

The term "melatonin" is used hereafter to designate both the actual melatonin and the chemical homologues or derivatives thereof being effective in a similar manner and as being defined above.

Description of The Preferred Embodiments

In a first preferred embodiment of the invention, the composition is in a form suitable for topical application for humans. Advantageously, this composition is in the form of a cream, ointment or lotion or another cosmetic liquid applicable externally. Such composition is then of a particular utility for preventing, attenuating or curing acne or for conditioning the skin to improve its appearance and texture.

Acne is a common inflammatory pilosebaceous disease characterized by comedones, papules, pustules, inflamed nodules, superficial pus-filled cysts, and, in extreme cases, canalizing and deep inflammed, sometimes purulent sacs. Its pathogenesis is complex; an interaction between hormones, keratinization, sebum and bacteria somehow determines the course and severity of the disease. The common form of acne is a feature of adolescence in both sexes, and androgen seems to be the essential factor, operating through stimulation of the sebaceous glands. Acne lesions predominate on the face but are also common on the neck, chest, upper back and shoulders.

The compositions in accordance with the present invention are particularly useful in the control of simple uncomplicated acne, such as, acne vulgaris. Acne vulgaris is a condition which involves the eruption of papules and pustules on an inflammatory base. The condition occurs primarily during puberty and adolescence due to overactive sebaceous apparatus which is believed to be affected by hormonal activity.

The other components of such compositions, can be the usual ones. It is advantageous to use compositions in which melatonin is associated with a lipophylic substance, both being dissolved in an appropriate solvant. For instance, the melatonin homologues may be used in the form of a solution in a water-ethanol mixture containing from 10% to 30% v/v or more of ethanol. However, the amount of active ingredients may be more or less depending on the specific conditions to be treated and the degree of treatment needed.

The compositions for acne and seborrhea control or skin conditioning can also be used in a therapeutic environment or application, in addition to the cosmetic uses which have been mentioned here above. Particularly, the composition of the invention, whether it be used locally or regionally in the form of a cream, ointment or lotion or of another form of composition, can also be used for the prevention or treatment of androgen or drug-induced acne or seborrhea, for instance, in patients subjected to androgenous hormone therapy in cancer therapy.

In addition, melatonin can be used alone or in conjunction with topical estrogens to enhance topical or endogenous estrogen action on the skin for the prevention and treatment of acne or seborrhea or for cosmetic effects related to improving the texture and physical appearance of aging skin.

The prime use of melatonin, alone or in conjunction with estrogens will be during adolescence when the oiliness of skin and the plugging of sebaceous glands results in acne vulgaris and seborrhea. Alternatively, alone or in conjunction with estrogen in pre or post menopausal women where dryness and loss of skin texture has been ascribed to the decline in estrogen. The estrogens may be applied separately or in combination with the melatonin.

An additional use for melatonin is by direct applica tion to the vaginal mucosa, alone or in conjunction with estrogen to restore the quality and secreting capacity of atrophic sexual skin in the vagina.

The topical application of melatonin, alone or in conjunction with estrogen (oral or parenteral administration) will improve the texture (elasticity and vascularity) of skin subject to atrophy seen in association with the loss of estrogen with aging.

Whatever the use of the topical composition or lotion, the concentration of the active compound therein should be sufficient for providing a good local absorption of the active ingredients.

The mechanism of melatonins local action is based on the observations that it causes a local increase of the binding activity of estrogen receptors and can ameliorate the action of testosterone on the skin through enhancement of the local effect of estrogen.

In regard to the above, the cause of acne vulgaris and seborrhea (increased sebum production, oily skin) can be due to an excess of testosterone or related androgenic hormones. Current treatment of acne vulgaris includes the topical or systemic use of agents that provide estrogen or suppress the production of

testosterone.

A suitable acne preparation may comprise the following:

Melatonin 10% in a colorless, greaseless lotion which contains water, aluminum hydroxide, isopropyl stearate, PEG-100 stearate, glyceryl stearate, cetyl alcohol, glycereth-26, isocetyl stearate, glycerin, dimethicone copolyol, sodium citrate, citric acid, methylparaben, propylparaben, fragrance. The preparation amongst the beneficial effects which the preparation provides are:

1.) helps heal and prevent acne pimples. Melatonin dries up and kills acne causing bacteria to help prevent new ones.

2.) helps absorb excess skin oil often associated with acne blemishes. The preparation preferably contains aluminum hydroxide, or other like materials, such as bentonite, which is a special oil absorbing ingredient that allows the lotion to absorb more excess skin oil than melatonin alone.

3.) helps the skin look fresh. Extra oil absorption helps the skin look less oily, more natural. If desired, the preparation can also contain sulfur and/or recorcinol or other known active ingredients to help heal the acne pimples. However, the additional ingredients may be applied separately.

The following publications describe the various methods of treating the acne condition:

- C. R. DARLEY et al., titled: "Circulating testosterone, sex hormone binding globulin and prolactin in women with late onset or persistent acne vulgaris". Br. J. Dermatology, 106: 517-522
- G. S. GINSBERG et al. (1981) titled: "Androgen abnormalities in acne vulgaris". Acta Dermatovener 61: 431-434.
- R. PALATSI, et al., (1978) titled: "Treatment of acne with cyproterone acetate and ethenyl estradiol". Acth Dermatovener 58: 449-454.
- J. H. CHERK (1980) titled: "Improvement of intractable acne in man following testosterone suppression using danazol". Cutis: 26: 393-394.
- J. B. SCHMIDT, J. SPONA (1983) titled: "Hormone receptors in normal skin and acne". Endocrinologica Experimentalis 17: 137-144, 1983.
- J. ZABEL: titled: "Uptake of $^3$H-testosterone in the skin of healthy women and in the skin of patients with acne vulgaris". Acth Histochem: 64: 243-248.

As previously mentioned, melatonin has been shown to have a direct effect on the metabolism of steroids in peripheral organs such as ovary, testes, adrenals and prostate. Thus, its action on the skin may be by cutaneous alteration in the metabolism of androgens, i.e., blocking the targeting effects of testosterone on sebaceous glands and hair follicles. Alternatively, melatonin may produce its therapeutic effect via an increase in estrogenic receptors in the skin and seborrheic glands. Melatonin can feminize the skin by increasing its capacity to respond to estrogen through an increase in cutaneous estrogen receptors or through an attenuation of androgen response.

The action of estrogen as a skin conditioner is enhanced by melatonin's local action in modulating the increase in estrogenic receptors. Thus melatonin's action on aging skin of postmenapausal women or aging males is mediated by enhancing local estrogenic effects. This is of value in restoring cosmetic quality to the skin (moisture, vascularity, elasticity) as well as restoring the local response of vaginal epithelium to estrogen.

More generally, the compositions of the invention (either in the form of lotions, creams, or ointments) of the invention are useful for the up-keeping of skin in good condition or for skin-regeneration, particularly in an aging population. The administration or use of melatonin will allow in particular the restoration of the skin functions including elasticity and moisture content. Even more generally melatonin is capable of controlling body odors by altering sebaceous secretions, the up-keeping of skin moisture and of all key factors associated with hormones involved with sebaceous or secretion which govern in part sexual attraction. The invention particularly includes perfume compositions deodorants or toiletries supplemented with melatonin.

In regard to the application of melatonin for the control of body hair, it is advantageous to use compositions in which melatonin is associated with a lipophilic substance, both being dissolved in an appropriate solvent. For instance, the melatonin homologues are used in the form of a solution in a water-ethanol mixture containing from 10% to 30% v/v or more of ethanol. Alternatively, it can be administered as a cream or ointment. However, the amount of active ingredients in the composition may be more or less depending on the specific conditions to be treated and the degree of treatment needed.

A second embodiment of the invention relates to compositions containing an effective amount of melatonin associated with a vehicle which makes said composition suitable for topical application for preventing hair fall or regeneration of hair to the extent where the hair roots or follicles are not yet fully degenerated or dead. That is, hair follicles which are sitll capable of sustaining hair growth and can be rejuvenated. As a matter of fact, it has been found that the definitive death of hair often takes place about 3 or 4 years after the actual fall. Therefore, under such circumstances, renewed growth of hair which is not

yet dead can even be observed. The other components of such compositions can be the usual ones. It is advantageous to use compositions in which melatonin is associated with a lipophylic substance, both being dissolved in an appropriate solvent. For instance, the melatonin homologues are used in the form of a solution in a water-ethanol mixture containing from 10 % to 30 % v/v or more of ethanol. However, the amount of active ingredients in the composition can be more or less depending upon the patient, the conditions of the scalp and the effect desired.

In accordance with the present invention, the compositions for hair control or regeneration can also be used in a therapeutical environment or application, in addition to the cosmetic uses which have been mentioned here above. Particularly, the composition of the invention, whether it be in the form of a lotion or of another form of composition, can also be used for the prevention or treatment of drug-induced or toxic alopecia, for instance in patients subjected to androgenous hormone therapy or cancer chemotherapy which, as it is well known, often causes temporary complete hair fall. The topical application of melatonin then both slows down the drug-induced alopecia, and/or favors the hair recovery when the therapy concerned is over.

Toxic alopecia is distinguishable from male-pattern baldness. Toxic alopecia is usually temporary and may follow a severe, often febrile illness. It may also be seen in myxedema, hypopituitarism, following pregnancy, and with some drugs. In such cases there is degeneration of the hair follicles which unless stimulated or rejuvenated would result in a permanent loss of the hair follicles.

Whatever the use of the topical composition or lotion, the concentration of the active compound therein should be sufficient for providing a good local absorption of the active principle. It is believed that melatonin, when so applied, causes a local increase of the binding activity of estrogen receptors and also neutralizes the local testosterone.

The topical application in a significant number of persons who used a melatonin lotion by topical head application, resulted in the complete interruption of the hair fall which they were experiencing previously. Melatonin was also found to act in them as a regulator of sebaceous glands.

Prior to the present invention, there have been no clinical or laboratory studies with regard to the topical application of melatonin to cutaneous areas.

The following tests were conducted utilizing ten male subjects suffering from hair loss and baldness, a preparation containing melatonin in a concentration of 1mg/ml in 30 % ethyl alcohol.

The solution of melatonin (Fluka AG, Buchs, Switzerland) was prepared containing 1mg/ml in 30% ethyl alcohol. The solution was kept in a dark bottle at 4 degrees C. The stability of melatonin in the solution was periodically checked by HPLC and no degradation or changes were observed in the course of three months.

A group of ten balding men with variable degree of hair loss, dander and sebaceous, fatty secretion of the scalp, aging 30 to 50 years, were treated daily in the evening (6 to 10 p.m.) with topic applications of melatonin by imbibition of the balding areas with melatonin solution combined with a light massage of the scalp for five minutes. The solution was left to dry and to act overnight. The treatment was continued for two weeks and then continued at three days intervals, twice a week for a further three months.

The effect of melatonin on scalp hair was evident after a few days of initiation of its topical application. Loss of hair first decreased and then completely stopped and permanently abrogated. Thinning, brittle fragile hair in balding areas grew vigorously and became identical to healthy normal hair in those areas maintaining hair growth. However, completely bald areas where hair had been lost for a long time (years), did not show any regeneration of hair growth. Also, in the areas treated with melatonin, dander, epitherlial desquamation and excessive sebaceous secretion disappeared completely and the scalp skin showed a clean, healthy, elastic appearance. Apparently, hair pigmentation was not affected. White, greying hair or black hair maintained their original color. When the treatment was interrupted for two weeks, a modest hair loss was observed which was immediately reversed by further continuation of melatonin application. No significant or alarming local or general toxic side effects of topic administration of melatonin have been observed at five months after beginning of the tests.

Even when administered per os at very high doses and for a long time, melatonin has been found to be singularly free of toxicity. Thus, topic use of melatonin does not constitute a danger if one considers that, in humans, pineal secretion of melatonin is a normal physiologic event with typical night-day periodicity.

This activity of melatonin on restoration and maintenance of scalp hair growth does thus constitute a valid "physiological" prophylaxis and therapy of the balding syndrome in humans suffering from toxic alopecia.

Although the invention relates primarily to the topical application of melatonin, it is understood that topical treatment may be in combination with oral or parenteral administration of melatonin in accordance with the present medical indications depending upon the patient and the severity of the condition to be treated.

By way of example, there may be mentioned merely by way of examples, relative concentrations of $10^{-4}$ per cent to 1 per cent in weight, of the melatonin or melatonin derivatives in lotions or other liquid solutions of, $10^{-4}$ per cent to 1 per cent in ointment compositions. Other suitable pharmaceutical carriers which may be utilized in the invention are described in F. W. Martin et al. "Remington's Pharmaceutical Sciences" 14th Ed. Mack Publishing Company, Easton, PA. 1965.

Generally, it is advantageous to apply or use the compositions of the invention in the evening and prior to going to sleep when melatonin endogenous production is reduces at a lower level. However, this is not a limiting condition for the use of melatonin compositions.

Nonetheless, when oral use is considered, effective daily dosages range from about 0.1 to about 100 mg/kg/day, the solid orally administrable compositions being included accordingly.

The following references together with other references which are mentioned hereinbefore relate to studies on various animal in the treatment of various conditions and are herein incorporated by reference.

REFERENCES


- Blask, D.E.: The pineal, and oncostatic gland? In: The Pineal Gland. ed. R. J. Reiter, Raven Press, New York, 1984, pp. 276-277.
- Bubenik, G.J.: Shift of seasonal cycle in white-tail deer by oral administration of melatonin. J. Exp. Zool. 225: 155-156, 1983.
- Burns, J.K.: Administration of melatonin to non-human primates and to women with breast carcinoma. J. Physiol. 229: 38-39, 1973.
- Danforth, J.R., Tamarkin, L., Do, R., Lippman, E.: Melatonin induced increase in cytoplasmic estrogen receptor activity in hamster uteri. Endocrinology 113: 81-85, 1983.
- Danforth, D.N., Jr., Tamarkin, L., Lippman, M.E.: Melatonin increases estrogen receptor binding activity of human breast cancer cells. Nature 305: 323-325, 1983.
- Fiske, V.M., Parker, K.L., Ulmer, R.A., Hoonow, H., Aziz, N.: Effect of melatonin alone or in combination with human chorionic gonadotropin or ovine luteinizing hormone on the in vitro secretion of estrogens on prosterone by granulosa cells of rats. Endocrinology 114,: 407-410, 1984.
- Heath, H.W., Lynch, G.R.: Intraspecific differences for melatonin-induced reproductive regression and the seasonal molt in peromyscus leucopus. Gen and Comp. endocrinol. 48: 289-295, 1982.
- Horst, H-J, Buck, A., Adam, K-U: Orally administered melatonin stimulates the 3 alpha/beta hydroxy steroid oxidoreductase but not the 5 alpha reductase in the ventral prostate and seminal vesicles of pinealectomized rats. Experientia 38: 968-970, 1982.
- Houssay, A.B., Pazo, J.H., Epper, C.E.: Effects of the pineal gland upon the hair cycles in mice. ACTA Physiol. Lat. Am. 202-205, 1966.
- Houssay, A.B., Pazo, J.H., Epper, C.E.: Effects of the pineal gland upon the hair cycles in mice. J. Invest. Dermatol. 47: 230-234, 1966.
- Moeller, H., Koz, A., Rodl, W., Frick, H-J., Gupta, D.: Role of pineal gland in the regulation of prostatic androgen receptors in pubertal and mature rats. Res. Exp. Med. 183: 157-165, 1983.
- Peat, F., Kinson, G.A.: Testicular steroidogenesis in vitro in the rat in response to blinding, pinealectomy and to the addition of melatonin. Steroids 17: 251-264, 1971.
- Petterborg, L.J., Reiter, R.J.: Effect of photoperiod and subcutaneous melatonin implants on the reproductive status of adult white footed mice (percomycus leucopus) U. Androl. 2: 222-224, 1981.
- Raikhlin, N.T., Kvetnoy, I.M., Tolkachev, V.N.: Melatonin may be synthesized in enterochromaffin cells. Nature 225: 344-345, 1975.
- Ralph, C.L.: Melatonin production by extra-pineal tissues. Adv. in the biosciences. ed.
- Rose, J., Stormshak, F., Oldfield, J, Adair, J.: Introduction of winter fur growth in mink (mustela vison) with melatonin. J. Animal Sci. 58: 57-61, 1984.
- Smythe, G.A., Lazarus, L.: Growth hormone response to melatonin in man. Science, 184: 1373-1374, 1974.
- Stetson, M.H., Rollag, M.D., Watson-Whitmyre, M, Tate-Ostroff, B.: The effect of daily injections and constant release implants of melatonin on the endogenous pineal rhythm in golden hamsters. Proc. Soc. Exp. Biol. Med. 174: 119-122, 1983.
- Stetson, M.H., Tay, D.E.: Time course of sensitivity of golden hamsters to melatonin injections throughout the day. Biol. of Reproduction 29: 432-438, 1983.
- Stetson, M.H., Watson-Whitmyre, M.: Physiology of the pineal and its hormone melatonin in animal reproduction in rodents. In: The Pineal Gland, ed. R. J. Reiter, Raven Press, New York, 1984, pp. 109-153.

- Tamarkin, L., Baird, C.J., Almeida, O.F.X.: Melatonin: A coordinating signal for mammalian reproduction. Science 227: 714-720, 1985.

**Claims**
**Claims for the following Contracting States : BE, CH, LI, DE, FR, GB, IT, LU, NL, SE**

**1.** A topical composition for use in the conditioning of the skin of a human host to improve its cosmetic and physical appearance, in the form of a cream, ointment, lotion or another cosmetic liquid, except solutions in water or aqueous injectable solutions, which comprises an active compound and a carrier suitable for said topical use on a site on the skin of said host, characterized in that said active compound is selected from
- melatonin,
- 5-methoxytryptamin,
- 5-methoxytryptophan,
- 5-methoxytryptophol,
- 5-methoxyindole-3-acetic acid,
- 6-hydroxy-melatonin.

**2.** The use of an active compound as indicated in claim 1 for the manufacture of a composition according to claim 1 which is suitable for the treatment of a human patient afflicted with acne, such as acne vulgaris, and wherein said carrier is a pharmaceutically acceptable carrier.

**3.** The use of an active compound as indicated in claim 1 for the manufacture of a composition according to claim 1 which is suitable for the control of seborrhea and wherein said carrier is a pharmaceutically acceptable carrier.

**4.** The use of an active compound as indicated in claim 1 for the manufacture of a composition according to claim 1 for use in the reduction of excessive hair fall or for use in the hair regeneration in the human host where the hair follicles are not degenerated and can be made to grow.

**5.** The use of an active compound as indicated in claim 1 for the manufacture of a composition according to claim 1 for use in the prevention or treatment of drug-induced or toxic alopecia.

**6.** The composition of claim 1 or the use according to any of claims 2 to 5 wherein said active compound is melatonin.

**7.** The composition of claim 1 of the use according to any of claims 2 to 6 wherein said active compound is present in a concentration ranging from $10^{-4}$ to 1 per cent by weight.

**8.** The composition or use of claims 6 and 7 wherein melatonin is present in a concentration ranging from $10^{-4}$ to 1 per cent by weight.

**9.** The use of an active compound selected from
- melatonin,
- 5-methoxytryptamin,
- 5-methoxytryptophan,
- 5-methoxytryptophol,
- 5-methoxyindole-3-acetic acid,
- 6-hydroxy-melatonin,

for the production of a topical composition for the conditioning of the skin of a human host to improve its cosmetic and physical appearance upon administering it to said human host.

**10.** The use of claim 9 for the production of a composition which is suitable for treating a patient afflicted with acne.

**11.** The use of claim 9 for the production of a composition which is suitable for the control of seborrhea.

**12.** The use of claim 9 for the production of a composition for causing the reduction of excessive hair fall or

is suitable for the hair regeneration in the human host where the hair follicles are not degenerated and can be made to grow.

**13.** The use of claim 9 for the production of a composition which is useful for the prevention or treatment of drug-induced or toxic alopecia.

**14.** The use of any of claims 9 to 13 wherein said active compound is melatonin.

**15.** The use of any of claims 9 to 14 wherein said composition is a lotion, solution, ointment or cream containing said melatonin in a concentration ranging from $10^{-4}$ to 1 per cent in weight.

**16.** The use of an active compound selected from
- melatonin,
- 5-methoxytryptamine,
- 5-methoxytryptophan,
- 5-methoxytryptophol,
- 5-methoxyindole-3-acetic acid,
- 6-hydroxy-melatonin,

for the production of a composition for use in humans for the reduction of excesive hair fall, for the regeneration of hair where the hair follicles are not degenerated and can be made to grow or for the prevention or treatment of drug-induced or toxic alopecia.

**17.** The use of an active compound selected from
- melatonin,
- 5-methoxytryptamin,
- 5-methoxytryptophan,
- 5-methoxytryptophol,
- 5-methoxyindole-3-acetic acid,
- 6-hydroxy-melatonin,

for the production of a composition for use in humans for the treatment of acne.

**18.** The use of claim 16 or 17 for the production of a composition which is administrable orally to said human host.

**19.** The use of any of claims 16 or 17 for the production of a composition which is administrable parenterally to said human host.

**Claims for the following Contracting State : AT**

**1.** A process for making topical composition for use in the conditioning of the skin of a human host to improve its cosmetic and physical appearance, in the form of a cream, ointment, lotion or another cosmetic liquid, except solutions in water or aqueous injectable solutions, which comprises mixing an active compound and a carrier suitable for said topical use on a site on the skin of said host, wherein said active compound is selected from
- melatonin,
- 5-methoxytryptamin,
- 5-methoxytryptophan,
- 5-methoxytryptophol,
- 5-methoxyindole-3-acetic acid.
- 6-hydroxy-melatonin,

**2.** The process of claim 1 wherein said carrier is a pharmaceutically acceptable carrier for use in compositions for the treatment of acne, such as acne vulgaris.

**3.** The process of claim 1 wherein said carrier is a carrier for use in compositions for the control of seborrhea.

**4.** The process of claim 1 wherein said carrier is a carrier for use in compositions for the reduction of

excessive hair fall or for use in the hair regeneration in the human host where the hair follicles are not degenerated and can be made to grow.

**5.** The process of claim 1 wherein said carrier is a carrier for use in the prevention or treatment of drug-induced or toxic alopecia.

**6.** The process of any of claims 1 to 5 wherein said active compound is melatonin.

**7.** The process of any of said claims 1 to 6 wherein said composition is made to contain said active compound in a concentration ranging from $10^{-4}$ to 1 per cent in weight.

**8.** The process of claim 7 wherein said active compound is melatonin.

**Revendications**
**Revendications pour les Etats contractants suivants : BE, CH, LI, DE, FR, GB, LU, NL, SE**

**1.** Une composition topique utile dans le conditionnement de la peau d'un hôte humain pour améliorer son aspect cosmétique et physique, sous la forme d'une crème, d'une pommade, d'une lotion ou d'un autre liquide cosmétique, à l'exception des solutions dans l'eau ou des solutions injectables aqueuses, qui comprend un composé actif et un support convenable pour cette utilisation topique sur un site de la peau de cet hôte, caractérisée en ce que ce composé actif est choisi parmi
- la mélatonine,
- la 5-méthoxytryptamine,
- le 5-méthoxytryptophane,
- le 5-méthoxytryptophol,
- l'acide 5-méthoxyindole-3-acétique,
- la 6-hydroxy-mélatonine,

**2.** L'utilisation d'un composé actif tel qu'indiqué dans la revendication 1 pour la fabrication d'une composition suivant la revendication 1, qui est convenable pour le traitement d'un patient humain souffrant d'acné, comme l'acné vulgaire, et dans laquelle ce support est un support acceptable du point de vue pharmaceutique.

**3.** L'utilisation d'un composé actif tel qu'indiqué dans la revendication 1 pour la fabrication d'une composition suivant la revendication 1, qui est convenable pour le contrôle de la séborrhée et dans laquelle ce support est un support acceptable du point de vue pharmaceutique.

**4.** L'utilisation d'un composé actif tel qu'indiqué dans la revendication 1 pour la fabrication d'une composition suivant la revendication 1, utilisable dans la réduction d'une chute excessive de cheveux ou utilisable dans la régénération de la chevelure d'un hôte humain chez lequel les follicules des cheveux ne sont pas dégénérés et dont la pousse peut être induite.

**5.** L'utilisation d'un composé actif tel qu'indiqué dans la revendication 1 pour la fabrication d'une composition suivant la revendication 1, utilisable dans la prévention ou le traitement d'une alopécie induite par des médicaments ou toxique.

**6.** La composition de la revendication 1 ou l'utilisation suivant l'une quelconque des revendications 2 à 5, dans lesquelles ce composé actif est la mélatonine.

**7.** La composition de la revendication 1 ou l'utilisation suivant l'une quelconque des revendications 2 à 6, dans lesquelles ce composé actif est présent en une concentration de l'ordre de $10^{-4}$ à 1% en poids.

**8.** La composition ou l'utilisation suivant les revendications 6 et 7, dans lesquelles la mélatonine est présente en une concentration de l'ordre de $10^{-4}$ à 1% en poids.

**9.** L'utilisation d'un composé actif choisi parmi:
- la mélatonine,
- la 5-méthoxytryptamine,

- le 5-méthoxytryptophane,
- le 5-méthoxytryptophol,
- l'acide 5-méthoxyindole-3-acétique,
- la 6-hydroxy-mélatonine.

pour la production d'une composition topique pour le conditionnement de la peau d'un hôte humain pour améliorer son aspect cosmétique et physique par administration de celle-ci à cet hôte humain.

**10.** L'utilisation suivant la revendication 9 pour la production d'une composition qui est convenable pour le traitement d'un patient souffrant d'acné.

**11.** L'utilisation suivant la revendication 9 pour la production d'une composition qui est convenable pour le contrôle de la séborrhée.

**12.** L'utilisation suivant la revendication 9 pour la production d'une composition pour provoquer la réduction d'une chute excessive de cheveux ou est convenable pour la régénération de la chevelure d'un hôte humain chez lequel les follicules des cheveux ne sont pas dégénérés et dont la pousse peut être induite.

**13.** L'utilisation suivant la revendication 9 pour la production d'une composition qui est convenable pour la prévention ou le traitement d'une alopécie induite par des médicaments ou toxique.

**14.** L'utilisation suivant l'une quelconque des revendications 9 à 13, dans laquelle ce composé actif est la mélatonine.

**15.** L'utilisation suivant l'une quelconque des revendications 9 à 14, dans laquelle cette composition est une lotion, une solution, une pommade ou une crème contenant cette mélatonine en une concentration de l'ordre de $10^{-4}$ à 1% en poids.

**16.** L'utilisation d'un composé actif choisi parmi:

- la mélatonine,
- la 5-méthoxytryptamine,
- le 5-méthoxytryptophane,
- le 5-méthoxytryptophol,
- l'acide 5-méthoxyindole-3-acétique,
- la 6-hydroxy-mélatonine.

pour la production d'une composition utile chez des humains pour la réduction d'une chute de cheveux excessive pour la régénération de la chevelure lorsque les follicules des cheveux ne sont pas dégénérés et dont la pousse peut être induite, ou pour la prévention ou le traitement d'alopécie induite par des médicaments ou toxique.

**17.** L'utilisation d'un composé actif choisi parmi:

- la mélatonine,
- la 5-méthoxytryptamine,
- le 5-méthoxytryptophane,
- le 5-méthoxytryptophol,
- l'acide 5-méthoxyindole-3-acétique,
- la 6-hydroxy-mélatonine.

pour la production d'une composition utile chez les humains pour le traitement de l'acné.

**18.** L'utilisation suivant les revendications 16 ou 17 pour la production d'une composition qui peut être administrée par voie orale à cet hôte humain.

**19.** L'utilisation suivant l'une quelconque des revendications 16 ou 17 pour la production d'une composition qui peut être administrée par voie parentérale à cet hôte humain.

**Revendications pour l'Etat contractant suivant : AT**

**1.** Un procédé pour préparer une composition topique utile dans le conditionnement de la peau d'un hôte

humain pour améliorer son aspect cosmétique et physique, sous la forme d'une crème, d'une pommade, d'une lotion ou d'un autre liquide cosmétique, à l'exception des solutions dans l'eau ou des solutions injectables aqueuses, qui comprend le mélange d'un composé actif et d'un support convenable pour cette utilisation topique sur un site de la peau de cet hôte, dans lequel ce composé actif est choisi parmi:
- la mélatonine,
- la 5-méthoxytryptamine,
- le 5-méthoxytryptophane,
- le 5-méthoxytryptophol,
- l'acide 5-méthoxyindole-3-acétique,
- la 6-hydroxy-mélatonine.

**2.** Le procédé suivant la revendication 1, dans lequel ce support est un support acceptable du point de vue pharmaceutique utilisable dans des compositions pour le traitement de l'acné, comme l'acné vulgaire.

**3.** Le procédé suivant la revendication 1, dans lequel ce support est un support utilisable dans des compositions pour le contrôle de la séborrhée.

**4.** Le procédé suivant la revendication 1, dans lequel ce support est un support utilisable dans des compositions pour la réduction d'une chute excessive de cheveux ou utilisables dans la régénération de la chevelure d'un hôte humain chez lequel les follicules des cheveux ne sont pas dégénérés et dont la pousse peut être induite.

**5.** Le procédé suivant la revendication 1, dans lequel ce support est un support utilisable dans la prévention ou le traitement d'une alopécie induite par des médicaments ou toxique.

**6.** Le procédé suivant l'une quelconque des revendications 1 à 5, dans lequel ce composé actif est la mélatonine.

**7.** Le procédé suivant l'une quelconque des revendications 1 à 6, dans lequel cette composition est faite de façon à contenir ce composé actif en une concentration de l'ordre de $10^{-4}$ à 1% en poids.

**8.** Le procédé suivant la revendication 7, dans lequel ce composé actif est la mélatonine.

**Patentansprüche**
**Patentansprüche für folgende Vertragsstaaten : BE, CH, LI, DE, FR, GB, LU, NL, SE**

**1.** Arzneimittel zur äußeren Anwendung für die Konditionierung von menschlicher Haut zur Verbesserung ihrer kosmetischen und physischen Erscheinung in Form einer Creme, Salbe, Lotion oder anderen kosmetischen Flüssigkeit, außer Lösungen in Wasser oder wäßrigen injizierbaren Lösungen, umfassend einen Wirkstoff und einen Träger, der für die äußere Anwendung auf einer menschlichen Hautstelle geeignet ist, **dadurch gekennzeichnet**, daß der Wirkstoff aus
- Melatonin,
- 5-Methoxytryptamin,
- 5-Methoxytryptophan,
- 5-Methoxytryptophol,
- 5-Methoxyindol-3-essigsäure, und
- 6-Hydroxy-melatonin

ausgewählt ist.

**2.** Verwendung eines in Anspruch 1 aufgeführten Wirkstoffs zur Herstellung eines Arzneimittels nach Anspruch 1, das zur Behandlung eines an Akne, wie Akne vulgaris, leidenden menschlichen Patienten geeignet ist und in dem der Träger ein pharmazeutisch verträglicher Träger ist.

**3.** Verwendung eines in Anspruch 1 aufgeführten Wirkstoffs zur Herstellung eines Arzneimittels nach Anspruch 1, das für die Seborrhoe-Bekämpfung geeignet ist und in dem der Träger ein pharmazeutisch verträglicher Träger ist.

**4.** Verwendung eines in Anspruch 1 aufgeführten Wirkstoffs zur Herstellung eines Arzneimittels nach Anspruch 1 zur Verwendung für die Verminderung von übermäßigem Haarausfall oder zur Verwendung für die Haarregeneration bei Menschen, deren Haarfollikel nicht degeneriert sind und zum Wachsen gebracht werden können.

**5.** Verwendung eines in Anspruch 1 augeführten Wirkstoffs zur Herstellung eines Arzneimittels nach Anspruch 1 zur Verwendung für die Vorbeugung oder Behandlung von durch Arzneimittel verursachter oder toxischer Alopecie.

**6.** Arzneimittel nach Anspruch 1 oder Verwendung nach einem der Ansprüche 2 bis 5, wobei der Wirkstoff Melatonin ist.

**7.** Arzneimittel nach Anspruch 1 oder Verwendung nach einem der Ansprüche 2 bis 6, wobei der Wirkstoff in einer Konzentration im Bereich von $10^{-4}$ bis 1 Gew.-% vorhanden ist.

**8.** Arzneimittel oder Verwendung nach den Ansprüchen 6 und 7, wobei Melatonin in einer Konzentration im Bereich von $10^{-4}$ bis 1 Gew.-% vorhanden ist.

**9.** Verwendung eines Wirkstoffs ausgewählt aus
- Melatonin,
- 5-Methoxytryptamin,
- 5-Methoxytryptophan,
- 5-Methoxytryptophol,
- 5-Methoxyindol-3-essigsäure, und
- 6-Hydroxy-melatonin

zur Herstellung eines Arzneimittels zur äußeren Anwendung für die Konditionierung von menschlicher Haut zur Verbesserung ihrer kosmetischen und physischen Erscheinung nach Verabreichung an einen Menschen.

**10.** Verwendung nach Anspruch 9 zur Herstellung eines Arzneimittels, das zur Behandlung eines an Akne leidenden Patienten geeignet ist.

**11.** Verwendung nach Anspruch 9 zur Herstellung eines Arzneimittels, das zur Seborrhoe-Bekämpfung geeignet ist.

**12.** Verwendung nach Anspruch 9 zur Herstellung eines Arzneimittels, das die Verminderung von übermäßigem Haarausfall herbeiführt oder für die Haarregeneration bei Menschen, deren Haarfollikel nicht degeneriert sind und zum Wachsen gebracht werden können, geeignet ist.

**13.** Verwendung nach Anspruch 9 zur Herstellung eines Arzneimittels, das zur Vorbeugung oder Behandlung von durch Arzneimittel verursachter oder toxischer Alopecie verwendbar ist.

**14.** Verwendung nach einem der Ansprüche 9 bis 13, wobei der Wirkstoff Melatonin ist.

**15.** Verwendung nach einem der Ansprüche 9 bis 14, wobei das Arzneimittel eine Lotion, Lösung, Salbe oder Creme ist, das Melatonin in einer Konzentration im Bereich von $10^{-4}$ bis 1 Gew.-% enthält.

**16.** Verwendung eines Wirkstoffs ausgewählt aus
- Melatonin,
- 5-Methoxytryptamin,
- 5-Methoxytryptophan,
- 5-Methoxytryptophol,
- 5-Methoxyindol-3-essigsäure, und
- 6-Hydroxy-melatonin

zur Herstellung eines Arzneimittels für die Verwendung in Menschen zur Verminderung von übermäßigem Haarausfall, zur Haarregeneration, wo die Haarfollikel nicht degeneriert sind und zum Wachsen gebracht werden können, oder zur Vorbeugung oder Behandlung von durch Arzneimittel induzierter oder toxischer Alopecie.

**17.** Verwendung eines Wirkstoffs ausgewählt aus
- Melatonin,
- 5-Methoxytryptamin,
- 5-Methoxytryptophan,
- 5-Methoxytryptophol,
- 5-Methoxyindol-3-essigsäure, und
- 6-Hydroxy-melatonin

zur Herstellung eines Arzneimittels zur Verwendung in der Behandlung von Akne bei Menschen.

**18.** Verwendung nach Anspruch 16 oder 17 zur Herstellung eines Arzneimittels, das oral an Menschen verabreichbar ist.

**19.** Verwendung nach einem der Ansprüche 16 oder 17 zur Herstellung eines Arzneimittels, das parenteral an Menschen verabreichbar ist.

**Patentansprüche für folgenden Vertragsstaat : AT**

**1.** Verfahren zur Herstellung eines Arzneimittels zur äußeren Anwendung für die Konditionierung von menschlicher Haut zur Verbesserung ihrer kosmetischen und physischen Erscheinung in Form einer Creme, Salbe, Lotion oder anderen kosmetischen Flüssigkeit, außer Lösungen in Wasser oder wäßrigen injizierbaren Lösungen, umfassend das Mischen eines Wirkstoffs und eines Trägers, der für die äußere Anwendung auf einer menschlichen Hautstelle geeignet ist, wobei der Wirkstoff aus
- Melatonin,
- 5-Methoxytryptamin,
- 5-Methoxytryptophan,
- 5-Methoxytryptophol,
- 5-Methoxyindol-3-essigsäure, und
- 6-Hydroxy-melatonin

ausgewählt ist.

**2.** Verfahren nach Anspruch 1, in dem der Träger ein pharmazeutisch verträglicher Träger zur Verwendung in Arzneimitteln für die Behandlung von Akne, wie Akne vulgaris, ist.

**3.** Verfahren nach Anspruch 1, in dem der Träger ein Träger für die Verwendung in Arzneimitteln zur Seborrhoe-Bekämpfung ist.

**4.** Verfahren nach Anspruch 1, in dem der Träger ein Träger für die Verwendung in Arzneimitteln zur Verminderung von übermäßigem Haarausfall oder zur Verwendung bei der Haarregeneration in Menschen, deren Haarfollikel nicht degeneriert sind und zum Wachsen gebracht werden können, ist.

**5.** Verfahren nach Anspruch 1, in dem der Träger ein Träger für die Verwendung zur Vorbeugung oder Behandlung von durch Arzneimittel verursachter oder toxischer Alopecie ist.

**6.** Verfahren nach einem der Ansprüche 1 bis 7, in dem der Wirkstoff Melatonin ist.

**7.** Verfahren nach einem der Ansprüche 1 bis 6, wobei das Arzneimittel so hergestellt wird, daß es den Wirkstoff in einer Konzentration im Bereich von $10^{-4}$ bis 1 Gew.-% enthält.

**8.** Verfahren nach Anspruch 7, in dem der Wirkstoff Melatonin ist.